# FASCICULE DE BREVET EUROPEEN

(11) **EP 1 581 282 B1**
(45) Date de publication et mention de la délivrance du brevet: **01.07.2009**
(21) Numéro de dépôt: 03799679.0
(22) Date de dépôt: 19.12.2003
(51) Int. Cl.: A61M 5/00, A61M 5/142

(54) **DISPOSITIF DE DISTRIBUTION POUR UN RESEAU D'ACHEMINEMENT DE FLUIDES MEDICAUX VERS UN PATIENT**
VORRICHTUNG ZUR MEDIZINISCHEN FLÜSSIGKEITSABGABE
DISTRIBUTION DEVICE FOR A SUPPLY NETWORK FOR SUPPLY OF MEDICAL FLUIDS TO A PATIENT

(30) Priorité: 20.12.2002 FR 0216432
(43) Date de publication de la demande: 05.10.2005
(73) Titulaire: SEDAT, 69540 Irigny (Rhône) (FR)
(72) Inventeur: DENOLLY, Pascal, F-38200 Jardin (FR)
(74) Mandataire: Blot, Philippe Robert Emile
(86) Numéro de dépôt international: PCT/FR2003/003854
(87) Numéro de publication internationale: WO 2004/058331

(56) Documents cités:
- EP-A- 1 074 221
- US-A- 3 859 985
- US-A- 4 645 496
- US-A1- 2002 123 737
- US-A1- 2002 151 854

## Description

La présente invention concerne un dispositif de distribution pour un réseau d'acheminement de fluides médicaux vers un patient. Elle concerne également un nécessaire d'injection d'un produit de contraste dans le corps humain.

Ce type de dispositif de distribution est notamment utilisé lors de procédures diagnostiques et thérapeutiques en radiologie interventionnelle. En effet, dans ce contexte particulier, le médecin est conduit à injecter dans les artères ou les veines d'un patient un produit de contraste, tout en contrôlant épisodiquement la pression de l'artère dans laquelle est introduit ce produit. A cet effet, le médecin crée un réseau d'acheminement du liquide de contraste jusqu'au patient à l'aide d'un ensemble de tuyaux souples reliés aux différentes tubulures ou voies du dispositif de distribution.

Durant ces procédures de radiologie interventionnelle, il est fréquemment nécessaire de nettoyer au moins une partie du dispositif de distribution, notamment la tubulure et le cathéter qui y est raccordé servant à acheminer le produit de contraste jusqu'au patient, en raison de la tendance de ce liquide à, au bout d'un certain temps d'immobilité, se figer et à se déposer sur les parois de la tubulure et du cathéter. A cet effet, les dispositifs de distribution comportent une ligne dite « de rinçage » ou ligne de « flush » dans laquelle circule sous pression une solution saline, ou sérum, destinée à entraîner le produit de contraste précédemment utilisé.

Un premier type de dispositif de distribution adapté à une telle application comporte une rampe d'au moins deux robinets trois voies montés en série. Chacun des robinets comporte un organe de commande manuel distinct.

Pour permettre l'injection du liquide de contraste, l'une des voies d'un premier robinet est reliée par une ligne pourvue d'un cathéter jusqu'au patient. Sur la voie restante du même robinet, est connecté un capteur de pression raccordé à la ligne de rinçage. Sur une première voie du second robinet est relié un réservoir de produit de contraste. Une seringue d'aspiration et d'injection est reliée à la seconde voie du second robinet.

Les deux robinets permettent de définir deux configuration de connexion entre les différents éléments du réseau, ces deux configurations étant utilisées successivement et de manière répétitive. Dans une première configuration, la seringue est reliée au réservoir de produit de contraste alors que le capteur de pression est relié au patient. Dans cette première configuration, du liquide de contraste peut être prélevé par le médecin grâce à la seringue et du liquide de rinçage circule jusqu'au patient. Dans la seconde configuration, le flacon contenant le liquide de contraste, ainsi que le capteur de pression et la ligne de rinçage sont isolés du réseau, alors que la seringue est mise en communication avec le patient. Dans cette seconde configuration, le médecin peut injecter le liquide de contraste au patient.

On comprend qu'avec un dispositif de distribution comportant une rampe regroupant deux robinets trois voies montés en série, le médecin doit agir sur les deux robinets chaque fois qu'il souhaite commuter le réseau entre les deux configurations utiles. Cette manipulation est source d'erreur, notamment puisque le médecin peut oublier d'agir sur l'un des deux robinets. De plus, le médecin doit utiliser ces deux mains, l'une pour maintenir le corps des robinets et l'autre pour agir successivement sur les deux organes de commande des robinets, rendant la manipulation relativement longue.

Un second type de dispositif de distribution adapté à l'application précitée, tel que celui décrit dans US-A-2002/0151854 et qui correspond au préambule de la revendication 1, comporte un distributeur comprenant un corps délimitant intérieurement une chambre de circulation fluidique dans laquelle débouche à la fois, une tubulure d'injection du produit de contraste, une tubulure de mesure de pression raccordée à une ligne de mesure de pression artérielle ou veineuse, et une tubulure sous pression, appelée parfois « tubulure patient », reliée à une ligne pourvue d'un cathéter pour acheminer le produit de contraste jusqu'à l'artère ou la veine du patient. Au moyen d'un tiroir mobile disposé dans la chambre, le médecin connecte sélectivement la tubulure sous pression soit avec la tubulure d'injection, soit avec la tubulure de mesure de pression. Pour permettre l'injection sous une pression suffisante, la tubulure d'injection est raccordée à l'extrémité distale d'un corps de seringue à l'intérieur duquel débouche une tubulure d'alimentation en produit de contraste reliée à un réservoir de ce produit. Pour permettre de rincer au moins la tubulure sous pression et la ligne qui y est raccordée, la ligne de rinçage est reliée à la tubulure de mesure de pression et est pourvue d'un organe de fermeture de type valve anti-retour de sorte que, au moyen d'une pompe d'entraînement placée le long de la ligne de rinçage, la tubulure sous pression reliée au patient peut être parcourue par la solution de rinçage lorsque cette tubulure est connectée à la tubulure de pression via la chambre à tiroir.

On comprend qu'avec un tel dispositif de distribution, le médecin doit à la fois veiller à la bonne position du tiroir mobile dans la chambre et au bon fonctionnement de la pompe d'entraînement de la solution saline chaque fois qu'il souhaite rincer le dispositif.

Bien que cette manipulation soit plus simple que celle d'un dispositif à plusieurs robinets, elle reste délicate, notamment puisque le médecin peut mal régler la pompe d'entraînement du fluide de rinçage et ainsi perturber les déplacements du tiroir dans la chambre de raccordement du distributeur. De plus, la manipulation de la pompe est relativement longue, d'autant plus qu'elle n'est pas nécessairement située à proximité du médecin tenant dans ses mains le dispositif de distribution à rincer.

L'invention a pour but de proposer un dispositif de distribution à distributeur tel que présenté ci-dessus, dont le rinçage est à la fois simplifié pour le médecin et amélioré, réduisant ainsi les risques d'erreur et les pertes de temps.

A cet effet, l'invention a pour objet un dispositif de distribution pour un réseau d'acheminement de fluides médicaux vers un patient, tel que défini à la revendication 1.

D'autres caractéristiques de ce dispositif, prises isolément ou selon toutes les combinaisons techniquement possibles, sont définies aux revendications dépendantes 2 à 10.

L'invention a également pour objet un nécessaire d'injection d'un produit de contraste dans le corps humain, tel que défini à la revendication 11.

L'invention sera mieux comprise à la lecture de la description qui va suivre, donnée uniquement à titre d'exemple et faite en se référant aux dessins sur lesquels :
- la Figure 1 est une vue partiellement en perspective d'un réseau d'acheminement de fluides comportant un dispositif de distribution selon l'invention ;
- la Figure 2 est une vue en coupe longitudinale du dispositif de distribution du réseau de la Figure 1 ;
- la Figure 3 est une vue en coupe selon le plan III-III indiqué sur la Figure 2 ;
- les Figures 4 et 5 sont des vues en élévation d'une partie d'extrémité du dispositif de la Figure 2 prises selon respectivement les flèches IV et V indiquées sur la Figure 3 ;
- la Figure 6 est une vue en coupe selon le plan VI-VI indiqué sur la Figure 2 ;
- les Figures 7, 8 et 9A sont des vues analogues à la Figure 2 illustrant trois étapes successives d'utilisation du dispositif de la Figure 2 ;
- les Figures 9B et 9C sont des vues analogues aux Figures 4 et 5 du dispositif pris à l'étape illustrée sur la Figure 9A ; et
- la Figure 10 est une vue analogue à la Figure 2 d'une variante du dispositif de distribution selon l'invention.

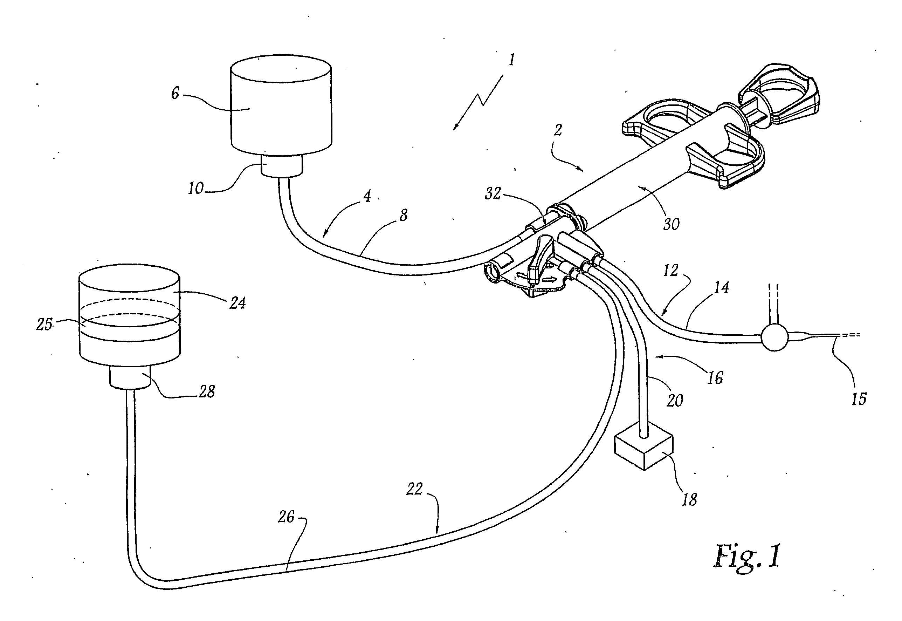

Sur la Figure 1 est représenté un réseau 1 d'acheminement de fluides médicaux utilisés lors de procédures en radiologie interventionnelle. Le réseau 1 comprend :
- un dispositif 2 de distribution des fluides ;
- une ligne 4 d'alimentation en un liquide de contraste comportant un réservoir 6 de ce liquide et un conduit souple 8 reliant le réservoir au dispositif de distribution 2 ; le conduit 8 est pourvu d'une chambre à goutte 10 comportant un filtre et une prise d'air ;
- une ligne 12 de sortie du liquide de contraste sous pression comportant un conduit souple 14 dont une extrémité est reliée au dispositif 2 et dont l'autre extrémité est pourvue d'un robinet trois voies ; l'une de ces voies est équipée d'un raccord luer, prolongé par un cathéter de coronographie 15 qui, en fonctionnement, est introduit dans l'artère ou la veine d'un patient ;
- une ligne 16 de mesure de pression artérielle ou veineuse comportant un capteur de pression 18 et un conduit souple 20 reliant ce capteur au dispositif 2 ; et
- une ligne de rinçage 22 comportant un réservoir flexible 24 de solution de rinçage, par exemple une solution saline, et un conduit 26 reliant ce réservoir 24 au dispositif 2 ; une sangle gonflable 25 ceinture le réservoir 24 de façon à, par élévation de la pression régnant dans cette sangle au moyen par exemple d'une poire de gonflage, maintenir le réservoir sous une pression suffisante pour entraîner la solution de rinçage le long de la ligne 22 ; le conduit 26 est pourvu d'une chambre à goutte 28.

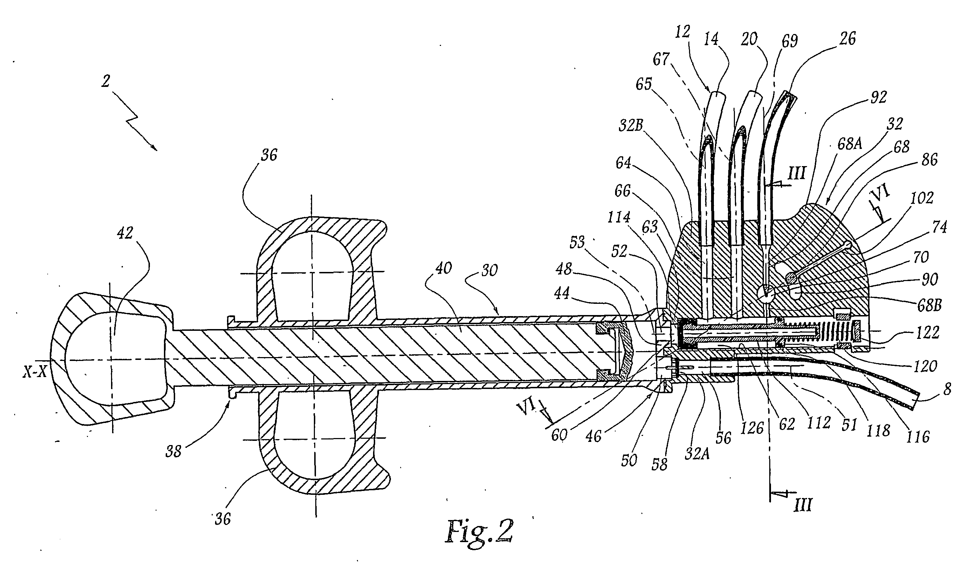

Le dispositif de distribution 2, représenté plus en détail sur les figures 2 à 6, comporte essentiellement un corps de seringue 30 et un distributeur 32.

Le corps de seringue 30 se présente sous la forme d'un cylindre creux d'axe X-X portant extérieurement une paire d'oreilles rigides 36 destinées à la prise en main du dispositif 2. Le corps cylindrique 30 comporte une extrémité proximale 38 à l'intérieur de laquelle est monté un piston 40 mobile suivant un mouvement de translation le long de l'axe X-X. L'extrémité proximale du piston 40 présente un évidement 42 d'actionnement manuel du piston et son extrémité distale est munie d'une tête coulissante 44 maintenant un contact étanche avec la face interne du corps de seringue.

Le corps 30 comporte une extrémité distale 46 formant une plaque 48 de fermeture du corps, percée de deux orifices cylindriques 50, 52 d'axes respectifs 51, 53 parallèles à l'axe X-X, et disposés de part et d'autre de cet axe X-X.

Le distributeur 32 est réalisé en un matériau rigide, par exemple en plastique moulé, et est solidarisé de façon étanche à l'extrémité distale 46 du corps de seringue 30, notamment par soudure par ultra-sons à la plaque de fermeture 48. A l'intérieur du distributeur 32 sont formés plusieurs orifices cylindriques ou tubulures.

Une première tubulure 56 est formée coaxialement à l'orifice 50, dans un premier corps 32A du distributeur 32 de forme extérieure essentiellement cylindrique. Cette tubulure est à son extrémité distale adaptée pour être raccordée au conduit 8 de la ligne 4 d'alimentation en produit de contraste. A son extrémité proximale, la tubulure 56 débouche dans l'orifice 50 et est équipée d'une valve en silicone 58 déformable élastiquement et adaptée pour autoriser la mise en communication de la tubulure 56 avec le volume intérieur du corps de seringue 30 lorsque la pression régnant dans ce corps de seringue est inférieure à celle régnant dans la tubulure 56.

Le distributeur 32 comporte un second corps 32B solidaire du premier corps 32A, ces deux corps étant formés d'une pièce d'un seul tenant. Dans ce corps 32B, une deuxième tubulure 60 est formée coaxialement à l'orifice 52 et relie ainsi directement le volume intérieur du corps de seringue 30 à une chambre cylindrique 62 ménagée dans le corps 32B, coaxialement à la tubulure 60. Le diamètre de cette chambre est plus grand que celui de la tubulure 60, de sorte qu'un épaulement 63 est formé.

Des tubulures 64, 66 et 68, d'axes respectifs 65, 67 et 69 parallèles entre eux, sont formées dans le corps 32B suivant une direction sensiblement perpendiculaire à l'axe X-X. Ces tubulures 64, 66 et 68 débouchent chacune, à une de leurs extrémités, directement dans la chambre 62 et, à leur autre extrémité, sont adaptées pour être raccordées respectivement au conduit 14 de la ligne 12 de sortie du liquide de contraste sous pression, au conduit 20 de la ligne 16 de mesure de pression et au conduit 26 de la ligne de rinçage 22.

Contrairement aux tubulures 64 et 66 qui mettent directement en communication la chambre 62 avec respectivement les lignes 12 et 16, la tubulure 68, qui présente dans sa partie courante un diamètre plus petit que celui des tubulures 64 et 66, comporte deux tronçons 68A, 68B séparés par un obturateur 70. Cet obturateur présente essentiellement une forme cylindrique d'axe Z-Z sensiblement perpendiculaire au plan contenant l'axe X-X et l'axe 69 de la tubulure 68. L'obturateur 70 est reçu dans un évidement cylindrique complémentaire 72 formé par le distributeur 32. La partie courante du cylindre formant cet obturateur est constitué d'un secteur 74 adapté pour fermer l'extrémité attenante du tronçon 68A.

L'obturateur 70 est mobile par rapport au distributeur 32 suivant un mouvement de rotation autour de l'axe Z-Z de façon à modifier la position angulaire du secteur 74 à l'intérieur du logement 72 et ainsi mettre en communication fluidique les deux parties 68A et 68B de la tubulure 68.

L'étanchéité du montage de l'obturateur 70 est assurée par le logement 72 fermé à une de ses extrémités et par un joint torique 76 interposé, à l'autre extrémité du logement 72, entre les parois du logement et l'obturateur.

Pour commander le déplacement en rotation de l'obturateur 70, le dispositif de distribution 2 comporte une manette de commande 80 à actionnement manuel. Cette manette comporte un corps 82 de forme essentiellement parallélépipédique, relié de manière fixe à l'obturateur 70 de façon à rendre la manette basculable autour de l'axe Z-Z par rapport au distributeur 32. Pour le dispositif représenté, l'obturateur et la manette sont formés d'un seul tenant.

Le corps 82 de la manette 80 comporte deux faces latérales opposées adaptées pour former des surfaces 84 d'appui digital lors de la sollicitation manuelle en basculement de la manette.

Sur sa face tournée vers le distributeur 32, le corps 82 de cette manette est pourvu d'un pion saillant cylindrique 86 reçu à l'intérieur d'une rainure de guidage 90 formée dans une platine 92 du corps 32B du distributeur 32. Cette rainure s'étend suivant un arc de cercle centré sur l'axe Z-Z, sur une longueur prédéterminée de façon que, lorsque le pion 86 atteint un fond de cette rainure, la manette 80 est suffisamment basculée autour de l'axe Z-Z pour dégager le secteur 74 de l'extrémité de la partie 68A de la tubulure 68.

Le dispositif de distribution 2 est en outre pourvu de moyens de rappel élastique de la manette 80 dans sa position de fermeture dans laquelle elle place angulairement le secteur 74 sensiblement dans l'axe 69 de la tubulure 68, comme représenté sur les figures 2 à 6. Ces moyens comportent une lame souple 94 reliée mécaniquement au corps 82 de la manette 80 en étant solidarisée au pion 86. Cette lame s'étend parallèlement à la face du corps 82 tournée vers la platine 92. Dans l'exemple représenté, la lame 94 est formée d'un seul tenant avec le pion 86 et le corps 82 de la manette.

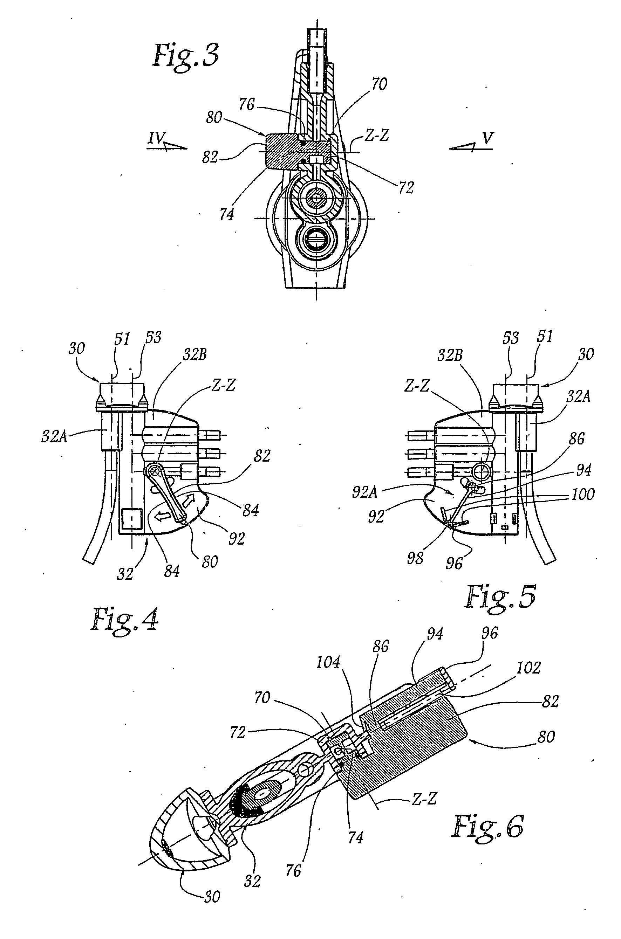

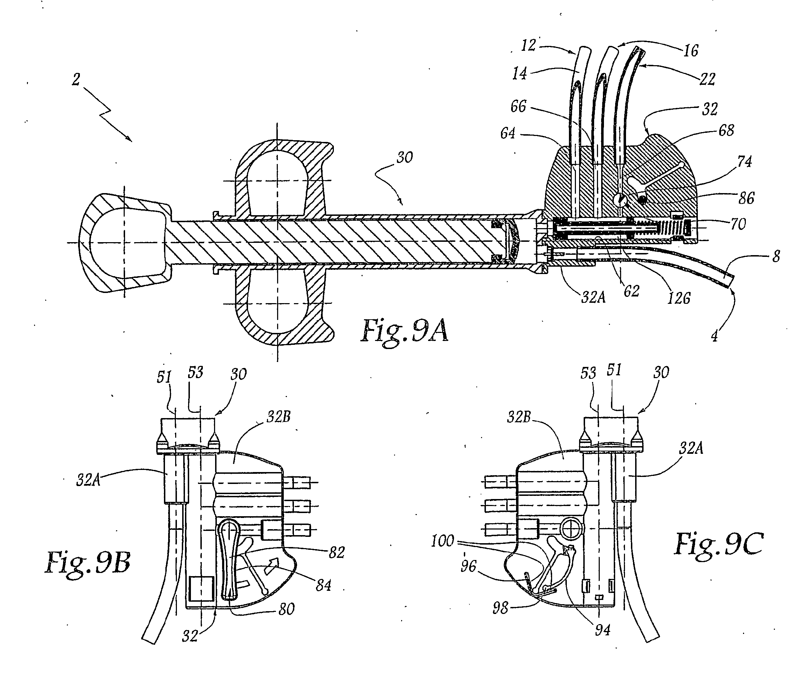

Les moyens de rappel élastique comportent également un rebord 96 en saillie de la face 92A de la platine 92 opposée à la manette 80. Ce rebord présente une section en forme de V dont la pointe reçoit intérieurement l'extrémité libre de la lame 94, comme représenté sur la Figure 5. Plus précisément, cette extrémité libre est formée d'une sphère 98 adaptée pour coopérer avec les faces en regard 100 du rebord 96 de façon à contraindre, par restriction du libre mouvement de cette sphère 98, le corps de la lame 94 à se déformer élastiquement en arc de cercle lorsque la manette 80 est basculée autour de l'axe Z-Z (figure 9C).

A des fins de montage de l'ensemble formé de l'obturateur 70, de la manette 80 et de la lame 94 sur le distributeur 32, une rainure 102 de forme complémentaire de la lame 94 et qui s'étend sensiblement suivant la bissectrice de l'angle formé par le V du rebord 96, est ménagée dans la platine 92 et débouche dans la rainure courbe 90, ce qui permet de mettre en place cet ensemble en faisant passer la lame 94 du côté de la platine 92 opposé à celui de la manette 80. Comme représenté sur la Figure 6, l'extrémité de la lame solidaire du pion 86 est pourvue d'un clip 104 de retenue de l'ensemble précité par rapport au distributeur 32, ce clip 104 étant déformé élastiquement lors de la mise en place de l'ensemble puis en appui sur la surface 92A de la platine. Dans l'exemple représenté, ce clip est formé d'un seul tenant avec la lame 94.

Le dispositif 2 comporte en outre pour connecter sélectivement les deuxième 60, troisième 64, quatrième 66 et cinquième 68 tubulures via la chambre 62, un tiroir 112 mobile en translation suivant l'axe 53 de la chambre 62, équipé à son extrémité proximale d'une tête 114 en contact étanche avec les parois de la chambre et à son extrémité distale d'un rebord annulaire 116 muni d'un joint d'étanchéité 118. La tête 114 et le rebord 116 sont écartés axialement d'au moins la distance séparant les tubulures 64 et 68.

Un ressort de compression 120 est interposé entre le rebord 116 et un couvercle rigide 122 solidarisé au corps 32B du distributeur, par exemple par clipsage. Le tiroir 112 forme de la sorte avec les parois de la chambre 62 d'une part un compartiment proximal 124, non représenté sur les figures 2 à 6, de volume variable selon la position du tiroir dans la chambre, et d'autre part entre la tête 114 et le rebord 116 un compartiment distal 126 de volume constant.

L'utilisation du réseau d'acheminement 1 et du dispositif 2 qu'il comporte est la suivante :

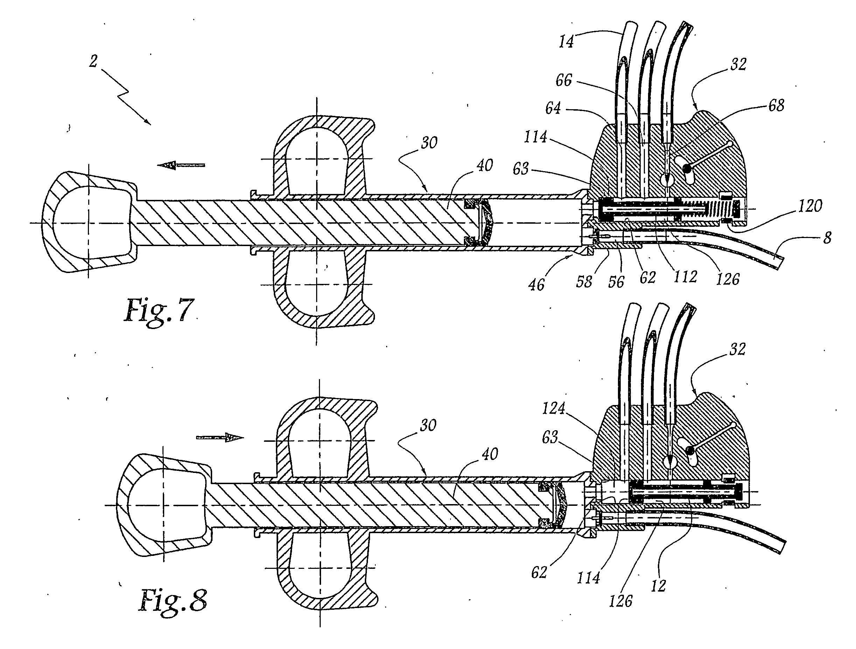

Alors que le cathéter 15 est introduit dans l'artère ou la veine d'un patient et que l'ensemble des éléments du réseau 1 sont convenablement raccordés comme sur la figure 1, le médecin met en oeuvre une première phase de remplissage du corps de seringue 30 à partir du réservoir 6 du liquide de contraste. A cet effet, comme représenté sur la figure 7, le piston 40 est déplacé par rapport au corps de seringue de façon à générer une dépression dans la partie distale 46 du corps de seringue. La différence de pression entre l'intérieur du corps de seringue et la première tubulure 56 entraîne la déformation de la valve 58 et le remplissage de la partie distale du corps de seringue par le liquide de contraste. Durant cette première phase, la tête 114 du tiroir 112 est maintenue en appui contre l'épaulement 63 par le ressort 120 de sorte que le volume du compartiment 124 est nul et que le cathéter 14 est mis en communication avec le capteur de pression 18, par la connexion de la tubulure 64 à la tubulure 66 via le compartiment 126 délimité par la chambre 62.

Pour procéder à l'injection proprement dite du liquide de contraste, le médecin, lors d'une seconde phase, établit une connexion entre le corps de seringue 30 et la tubulure 64. Pour cela, comme représenté sur la figure 8, il déplace le piston 40 en direction du distributeur 32 de façon à augmenter la pression du liquide de contraste jusqu'à ce que cette dernière pousse le tiroir 112 suivant la même direction, connectant alors les tubulures 60 et 64 via le compartiment 124. Le produit de contraste circule alors jusqu'au patient.

Durant cette seconde phase, le capteur de pression 18 est isolé de la tubulure sous pression 64 par la tête 114 du tiroir 112, et le réservoir de liquide de contraste 6 est isolé du corps de seringue 30 par la valve 58 non déformée.

Une fois que le piston 40 est parvenu en fin de course, le médecin recule le piston vers la partie proximale du corps de seringue. Le ressort de compression 112 repousse alors automatiquement le tiroir en direction du corps de seringue de façon à ce que les tubulures 64 et 66 soient de nouveau connectées par la chambre 62 comme dans la première phase, permettant au médecin de connaître la pression qui règne dans la ligne sous pression 12, c'est-à-dire la pression artérielle ou veineuse du patient.

Au bout d'un certain temps d'immobilité du liquide contraste dans la tubulure 64 et la ligne sous pression 12, il est nécessaire de les rincer pour éviter que le produit de contraste ne se fige et/ou ne se fixe sur les parois de ces éléments, notamment dans leurs parties de faible diamètre. Pour cela, dans une troisième phase d'utilisation représentée sur les figures 9A à 9C, le médecin, qui tient déjà le corps de seringue 30 d'une main, actionne par son autre main la manette de commande 80 en la déplaçant suivant un mouvement de basculement autour de l'axe Z-Z, dans un sens ou dans un autre, jusqu'à ce qu'elle occupe une position extrême dans laquelle le pion 86 est situé dans l'un des fonds de la rainure 90.

Le basculement et le maintien en place de la manette dans cet état basculé par le médecin provoquent la rotation de l'obturateur 70, ce qui permet le libre passage de la solution de rinçage depuis le réservoir 24 jusqu'au compartiment 126, comme représenté sur les figures 9A, 9B et 9C.

La solution de rinçage circule alors, via la chambre 62, dans les tubulures 64 et 66, et donc dans les lignes 12 et 16. Le liquide de contraste précédemment utilisé est entraîné par la solution saline, et d'éventuelles bulles d'air retenues le long de la ligne de pression 16 sont évacuées pour réduire le risque de mauvaises mesures de pression.

Une fois le rinçage effectué, le médecin relâche la manette 80 qui reprend sa position initiale par coopération de la lame 94 et du rebord 96 rappelant élastiquement la manette, et par là replaçant l'obturateur 70 en position de fermeture de la tubulure de rinçage 68. Le dispositif 2 se retrouve alors dans son état initial.

Le dispositif de distribution selon l'invention permet ainsi au médecin de rincer facilement l'essentiel du dispositif, en un temps limité et sans avoir à actionner des systèmes extérieurs au dispositif que tient en main le médecin, tel qu'une pompe déportée. Le fait de rendre le distributeur 32 solidaire du corps de seringue 30 permet d'obtenir un dispositif compact, même pour un dispositif à injection manuel comme décrit jusqu'ici. Dans la mesure où la tubulure de rinçage débouche directement dans la chambre 62, sans interposition de par exemple un raccord, la formation de bulles dans le réseau lors de son rinçage est fortement limitée.

En variante non représentée, la géométrie de la face périphérique du secteur de fermeture 74 de l'obturateur 70 est aménagée de façon à ce qu'au moins un faible débit de fluide de rinçage soit maintenu, quelle que soit la position de l'obturateur 70. De la sorte, même lorsque la manette 80 n'est pas actionnée par le médecin, une faible quantité de solution de rinçage s'écoule en continu dans le compartiment 126 de la chambre 62. De la sorte, l'importance d'un éventuel reflux de liquide de contraste et/ou de sang lors du retour de la tête 114 du tiroir 112 contre l'épaulement 63, c'est-à-dire en toute fin de la phase d'injection, est limitée.

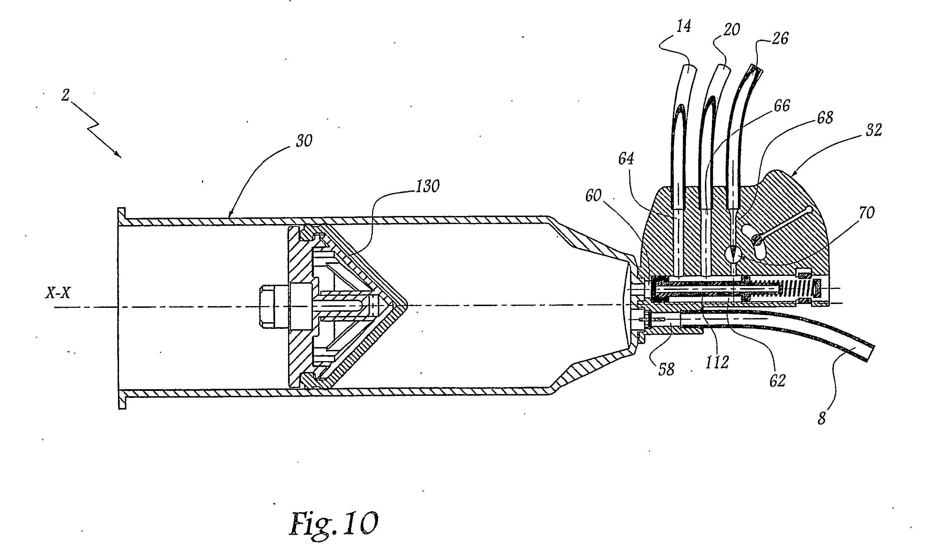

Sur la figure 10 est représentée une variante du dispositif 2 destinée à intégrer celui-ci à un appareil de sollicitation motorisé.

La différence essentielle entre le dispositif de la figure 10 et celui des figures précédentes tient dans le corps de seringue 30 et le piston 40. Dans cette variante, les oreilles de préhension du corps de seringue 30 sont absentes et le piston est constitué d'une tête 130 adaptée pour être fixée à une tige non représentée à entraînement motorisé, notamment par un système automatisé.

Une telle variante permet d'injecter un liquide de contraste sous des pressions plus élevées que celles atteintes par le dispositif manuel décrit précédemment.

Divers aménagements et variantes au dispositif de distribution et au réseau d'acheminement décrits jusqu'ici sont envisageables :
- le distributeur 32 est solidarisable à l'extrémité distale 46 du corps de seringue 30 par tous moyens garantissant la solidité et l'étanchéité d'une telle liaison, par exemple par collage ou en formant ces pièces d'un seul tenant notamment par moulage ;
- à la différence de l'exemple représenté, le corps 32A à l'intérieur duquel est formée la tubulure 66 reliant la ligne d'alimentation en liquide de contraste 4 ou corps de seringue 30 n'est pas nécessairement formé d'un seul tenant avec le corps 32B à l'intérieur duquel est formée la chambre de connexion 62, mais peut être mécaniquement indépendant de ce corps 32B, en étant par exemple soit formé d'un seul tenant par la partie d'extrémité distale du corps de seringue, soit en étant solidarisé à l'extrémité distale du corps de seringue par des moyens de liaison analogues à ceux décrits précédemment entre le corps de seringue 30 et le distributeur 32 ;
- la valve déformable 58 est remplaçable par un clapet à bille sensible à la différence de pressions régnant de part et d'autre du clapet ; et/ou
- le ressort 120 de rappel du tiroir coulissant 112 est remplaçable par une lame élastique, par exemple venue de matière avec le corps 32B du distributeur 32.

## Revendications

1. Dispositif de distribution pour un réseau (1) d'acheminement de fluides médicaux vers un patient, du type comportant :
- un corps de seringue (30),
- une tubulure (56) d'alimentation en un fluide médical actif, débouchant dans le corps de seringue (30) et destinée à être reliée à un réservoir (6) dudit fluide actif,
- un distributeur (32) comportant un corps (32B), à l'intérieur duquel est délimitée une chambre de circulation fluidique (62) et comportant, à l'intérieur de la chambre (62), un tiroir (112) mobile par rapport au corps (32B) du distributeur et un organe élastique (120) interposé entre le tiroir (112) et une partie fixe (122) de ce corps,
- une tubulure (60) d'injection du fluide actif, distincte de la tubulure d'alimentation (56), reliée à une extrémité distale (46) du corps de seringue (30) et débouchant dans la chambre (62) du distributeur,
- une tubulure sous pression (64) destinée à être reliée jusqu'au patient par une ligne sous pression (12) du réseau (1) et débouchant dans la chambre (62) du distributeur, et
- une tubulure de mesure de pression (66) destinée à être reliée à une ligne (16) de mesure de pression du réseau (1) et débouchant dans la chambre (62) du distributeur, lequel distributeur (32) est adapté pour connecter automatiquement, via ladite chambre, la tubulure sous pression (64) avec l'une seulement de la tubulure d'injection (60) et de la tubulure de mesure de pression (66), sous l'action de la pression du fluide actif et de l'organe élastique (120),
**caractérisé en ce que** le dispositif comporte en outre une tubulure de rinçage (68) distincte des autres tubulures (56, 60, 64, 66) du dispositif, formée dans le corps (32B) du distributeur (32) et comprenant un premier tronçon (68A) destiné à être relié à un réservoir (24) d'un fluide médical de rinçage et un second tronçon (68B) débouchant directement dans la chambre (62) du distributeur (32), laquelle tubulure de rinçage (68) est munie d'une vanne (70, 80) équipée d'un obturateur (70) disposé entre les premier et second tronçons (68A, 68B) de la tubulure de rinçage et déplaçable manuellement entre une position de fermeture au moins partielle de la tubulure de rinçage et une position de libre communication de la tubulure de rinçage (68) avec ladite chambre (62), le distributeur (32) étant adapté pour connecter, via ladite chambre (62), la tubulure de rinçage avec la tubulure sous pression (64) et la tubulure de mesure de pression (66),
et **en ce que** le tiroir (112) forme, avec les parois de la chambre (62), un compartiment (126), par lequel le fluide actif circule entre la tubulure sous pression (64) et la tubulure de mesure de pression (66) lorsque celles-ci sont connectées l'une avec l'autre, et par lequel le fluide de rinçage circule entre la tubulure de rinçage (68) et la tubulure de mesure de pression (66) lorsque celles-ci sont connectées 4l'une à l'autre.

2. Dispositif suivant la revendication 1, **caractérisé en ce que** la vanne (70, 80) de la tubulure de rinçage (68) est portée par le corps (32B) du distributeur (32).

3. Dispositif suivant la revendication 1 ou 2, **caractérisé en ce que** la vanne (70, 80) est montée à rotation autour d'un axe (Z-Z) orienté transversalement à la tubulure de rinçage (68).

4. Dispositif suivant l'une quelconque des revendications précédentes, **caractérisé en ce que** la vanne de la tubulure de rinçage (68) comporte un obturateur (70) de cette tubulure et une manette de commande manuelle (80), l'obturateur et la manette étant à la fois reliés mécaniquement l'un à l'autre et mobiles par rapport au corps (32B) du distributeur (32).

5. Dispositif suivant la revendication 4, **caractérisé en ce que** l'obturateur (70) comporte un secteur de cylindre (74).

6. Dispositif suivant l'une quelconque des revendications précédentes, **caractérisé en ce qu'**il comporte des moyens (94, 96) de rappel élastique de la vanne (70, 80) dans sa position de fermeture.

7. Dispositif suivant la revendication 6, **caractérisé en ce que** les moyens de rappel comportent une lame souple (94) en appui sur le corps (32B) du distributeur (32) et reliée mécaniquement à la vanne (70, 80) de la tubulure de rinçage (68).

8. Dispositif suivant l'une quelconque des revendications précédentes, **caractérisé en ce que** le corps (32B) du distributeur (32) est solidarisé de façon étanche au corps de seringue (30).

9. Dispositif suivant l'une quelconque des revendications précédentes, **caractérisé en ce que** la tubulure (56) d'alimentation en le premier fluide médical actif est délimitée par le distributeur (32).

10. Dispositif suivant l'une quelconque des revendications précédentes, **caractérisé en ce que** les tubulures d'alimentation (56) et d'injection (60) du fluide médical actif s'étendent suivant des directions sensiblement parallèles.

11. Nécessaire d'injection d'un produit de contraste dans le corps humain, **caractérisé en ce qu'**il comporte :
- un dispositif de distribution (2) selon l'une quelconque des revendications précédentes,
- une ligne (4) d'alimentation en produit de contraste comportant un conduit souple (8) équipé d'une chambre à goutte (10) et adapté pour être raccordé à une extrémité à un réservoir (6) de liquide de contraste et à son autre extrémité à la tubulure d'alimentation (56) du dispositif de distribution (2),
- une ligne sous pression (12) comportant à une extrémité un cathéter de coronographie (15) destiné à être introduit dans le corps du patient et adaptée pour être raccordée à son autre extrémité à la tubulure sous pression (64) du dispositif de distribution (2),
- une ligne (16) de mesure de pression comportant un conduit (20) équipé d'un capteur de pression (18) et adapté pour être raccordé à la tubulure de mesure de pression (66) du dispositif de distribution (2), et
- une ligne de rinçage (22) comportant un conduit souple (26) muni d'une chambre à goutte (28) et adapté pour être raccordé à une extrémité à un réservoir (24) d'une solution de rinçage et à son autre extrémité à la tubulure de rinçage (68) du dispositif de distribution (2).

## Claims

1. A distribution device for a system (1) for delivery of medical fluids to a patient, of the type comprising:
- a syringe body (30),
- a feed tube (56) for an active medical fluid, opening into the syringe body (30) and designed to be connected to a reservoir (6) for the said active fluid,
- a distributor (32) comprising a body (32B) within which there is bounded a chamber (62) for fluid circulation and comprising within the chamber (62) a slide (112) which can move with respect to the distributor and a resilient member (120) located between the slide (112) and a fixed part (122) of that body,
- a tube (60) for the injection of this active fluid connected to a distal extremity (46) of the syringe body (30) and opening into the chamber (62) of the distributor,
- a pressurised tube (64) designed to be connected to the patient through a pressurised line (12) of the system (1) and opening into the chamber (62) of the distributor, and
- a pressure measurement tube (66) designed to be connected to a line (16) for measuring the pressure of the system (1) and opening into the chamber (62) of the distributor, this distributor being designed to provide an automatic connection via the said chamber between the pressurised tube (64) and only one of the injection tubes (60) and the pressure measurement tube (66) through the action of the pressure of the medical fluid and the resilient member (120) **characterised in that** the device also comprises a flush tube (68) which is separate from the other tubes (56, 60, 64, 66) of the device formed in the body (32B) of the distributor (32) and comprising a first section (68A) which is designed to be connected to a reservoir (24) for a flush medical fluid and a second section (68B) opening directly into the chamber (62) of the distributor (32), the said flush tube (68) being fitted with a valve (70, 80) equipped with a plug (70) located between the first and second sections (68A, 68B) of the flush tube which can be moved manually between a position in which it at least partly closes the flush tube and a position in which the flush tube (68) is in free communication with the said chamber (62), the distributor (32) being designed to connect the flush tube with at least the pressurised tube (64) and the pressure measurement tube (66) via the said chamber (62),
and **in that** the slide (112) together with the walls of the chamber (62) forms a compartment (126) through which the active fluid circulates between the pressurised tube (64) and the pressure measurement tube (66) when these are connected to each other, and through which the flush fluid circulates between the flush tube (68) and the pressure measurement tube (66) when these are connected together.

2. A device according to claim 1, **characterised in that** the valve (70, 80) of the flush tube (68) is supported by the body (32B) of the distributor (32).

3. A device according to claim 1 or 2, **characterised in that** the valve (70, 80) is mounted so as to rotate about an axis (Z-Z) orientated transversely to the flush tube (68).

4. A device according to any of the preceding claims, **characterised in that** the valve in the flush tube (68) comprises a plug (70) for that tube and a manual control lever (80), the plug and the handle being both connected mechanically to each other and capable of movement with respect to the body (32B) of the distributor (32).

5. A device according to claim 4, **characterised in that** the plug (70) comprises a sector of a cylinder (74).

6. A device according to any one of the preceding claims, **characterised in that** it comprises means (94, 96) for resiliently returning the valve (70, 80) into its closed position.

7. A device according to claim 6, **characterised in that** the return means comprise a flexible blade (94) bearing against the body (32B) of the distributor (32) and mechanically connected to the valve (70, 80) of the flush tube (68).

8. A device according to any one of the preceding claims, **characterised in that** the body (32B) of the distributor (32) is made of one piece with the body of the syringe (30) in a leaktight manner.

9. A device according to any one of the preceding claims, **characterised in that** the tube (56) feeding the first active medical fluid is bounded by the distributor (32).

10. A device according to any one of the preceding claims, **characterised in that** the feed tube (56) and the injection tube (60) for the active medical fluid extend in substantially parallel directions.

11. A kit for the injection of a contrast product into the human body, **characterised in that** it comprises:
- a distribution device (2) according to any one of the preceding claims,
- a feed line (4) for contrast product comprising a flexible conduit (8) fitted with a drip chamber (10) and designed to be connected at one extremity to a reservoir (6) for contrast fluid and at its other extremity to the feed tube (56) of the distribution device (2),
- a pressurised line (12) comprising at one extremity a coronarography catheter (15) designed to be inserted into the patient's body and designed to be connected at its other extremity the pressurised tube (64) of the distribution device (2),
- a pressure measurement line (16) incorporating a conduit (20) fitted with a pressure sensor (18) and designed to be connected to the pressure measurement tube (66) of the distribution device (2), and
- a flush line (22) comprising a flexible conduit (26) fitted with a drip chamber (28) and designed to be connected at one extremity to a reservoir (24) for a flush solution and at its other extremity to the flush tube (68) of the distribution device (2).

## Patentansprüche

1. Verteilungsvorrichtung für ein Netz (1) zum Transportieren von medizinischen Fluiden zu einem Patienten, die Folgendes umfasst:
- einen Spritzenkörper (30),
- einen Anschlussstutzen (56) zum Zuführen eines aktiven medizinischen Fluids, der in den Spritzenkörper (30) mündet und der für eine Verbindung mit einem Reservoir (6) des genannten aktiven Fluids bestimmt ist,
- einen Verteiler (32), der ein Gehäuse (32B) umfasst, in dessen Innerem eine Fluidumlaufkammer (62) angeordnet ist und der im Innern der Kammer (62) einen in Bezug auf das Gehäuse (32B) des Verteilers beweglichen Schieber (112) und einen Federmechanismus (120) aufweist, der sich zwischen dem Schieber (112) und einem festen Teil (122) dieses Gehäuses befindet,
- einen Anschlussstutzen (60) zum Injizieren des aktiven Fluids, der vom Zuführungsanschlussstutzen (56) getrennt und mit einem distalen Ende (46) des Spritzenkörpers (30) verbunden ist und in die Kammer (62) des Verteilers mündet,
- einen Druckanschlussstutzen (64) zum Verbinden mit dem Patienten über eine Druckleitung (12) des Netzes (1), der in die Kammer (62) des Verteilers mündet, und
- einen Druckmessanschlussstutzen (66) zum Verbinden mit einer Druckmessleitung (16) des Netzes, der in die Kammer (62) des Verteilers mündet, wobei der Verteiler (32) so gestaltet ist, dass er den Druckanschlussstutzen (64) über die genannte Kammer automatisch entweder mit dem Injektionsanschlussstutzen (60) oder mit dem Druckmessanschlussstutzen (66) unter der Wirkung des Drucks des aktiven Fluids und des Federmechanismus (120) verbindet,
**dadurch gekennzeichnet, dass** die Vorrichtung darüber hinaus einen Spülanschlussstutzen (68) hat, der von den anderen Anschlussstutzen (56, 60, 64, 66) der Vorrichtung getrennt und im Körper (32B) des Verteilers (32) ausgebildet ist und einen ersten Abschnitt (68A) zum Verbinden mit einem Reservoir (24) eines medizinischen Spülfluids und einen zweiten Abschnitt (68B) aufweist, der direkt in die Kammer (62) des Verteilers (32) mündet, wobei der Spülanschlussstutzen (68) mit einem Ventil (70, 80) mit einer Absperrvorrichtung (70) versehen ist, die sich zwischen dem ersten und dem zweiten Abschnitt (68A, 68B) des Spülanschlussstutzens befindet und manuell zwischen einer wenigstens teilweisen Absperrposition des Spülanschlussstutzens und einer Position einer offenen Verbindung zwischen dem Spülanschlussstutzen (68) und der genannten Kammer (62) bewegt werden kann, wobei der Verteiler (32) zum Verbinden des Spülanachlussstutzens über die genannte Kammer (62) mit dem Druckanschlussstutzen (64) und dem Druckmessanschlussstutzen (66) gestaltet ist,
und **dadurch**, dass der Schieber (112) mit den Wänden der Kammer (62) ein Fach (126) bildet, durch das das aktive Fluid zwischen dem Druckanschlussstutzen (64) und dem Druckmessanschlussstutzen (66) umläuft, wenn diese miteinander verbunden sind, und über das das Spülfluid zwischen dem Spülanschlussstutzen (68) und dem Druckmessanschlussstutzen (66) umläuft, wenn diese miteinander verbunden sind.

2. Vorrichtung nach Anspruch 1, **dadurch gekennzeichnet, dass** das Ventil (70, 80) des Spülanschlussstutzens (68) vom Körper (32B) des Verteilers (32) getragen wird.

3. Vorrichtung nach Anspruch 1 oder 2, **dadurch gekennzeichnet, dass** das Ventil (70, 80) um eine transversal zum Spülanschlussstutzen (68) orientierte Achse (Z-Z) drehbar montiert ist.

4. Vorrichtung nach einem der vorherigen Ansprüche, **dadurch gekennzeichnet, dass** das Ventil (70, 80) des Spülanschluszetutzens (68) eine Absperrvorrichtung (70) dieses Anschlussstutzens und einen Handsteuergriff (80) aufweist, wobei die Absperrvorrichtung und der Steuergriff gleichzeitig mechanisch miteinander verbunden und mit Bezug auf den Körper (32B) des Verteilers (32) beweglich sind.

5. Vorrichtung nach Anspruch 4, **dadurch gekennzeichnet, dass** die Absperrvorrichtung (70) einen Zylindersektor (74) aufweist.

6. Vorrichtung nach einem der vorherigen Ansprüche, **dadurch gekennzeichnet, dass** sie Mittel (94, 96) zum federnden Zurückstellen des Ventils (70, 80) in seine Verschlussposition aufweist.

7. Vorrichtung nach Anspruch 6, **dadurch gekennzeichnet, dass** die Rückstellmittel eine biegsame Lamelle (94) aufweisen, die sich am Körper (32B) des Verteilers (32) abstützt und mechanisch mit dem Ventil (70, 80) des Spülanschlussstutzens (68) verbunden ist.

8. Vorrichtung nach einem der vorherigen Ansprüche, **dadurch gekennzeichnet, dass** der Körper (32B) des Verteilers (32) auf dichtende Weise fest mit dem Spritzenkörper (30) verbunden ist.

9. Vorrichtung nach einem der vorherigen Ansprüche, **dadurch gekennzeichnet, dass** der Anschlussstutzen zum Zuführen des ersten aktiven medizinischen Fluids durch den Verteiler (32) begrenzt wird.

10. Vorrichtung nach einem der vorherigen Ansprüche, **dadurch gekennzeichnet, dass** die Anschlussstutzen zum Zuführen (56) und Injizieren (60) des aktiven medizinischen Fluids im Wesentlichen parallel zueinander verlaufen.

11. Besteck zum Injizieren eines Kontrastmittels in einen menschlichen Körper, **dadurch gekennzeichnet, dass** es Folgendes umfasst:
- eine Verteilungsvorrichtung (2) nach einem der vorherigen Ansprüche,
- eine Leitung (4) zum Zuführen von Kontrastmittel, die einen biegsamen Conduit (8) umfasst, der mit einer Tropfenkammer (10) ausgestattet und so gestaltet ist, dass ein Ende am Kontrastflüssigkeitsreservoir (5) und das andere Ende am Zuführungsanschlussstutzen (56) der Verteilungsvorrichtung (2) angeschlossen werden kann,
- eine Druckleitung (12), die an einem Ende einen Koronarangiografie-Katheter (15) zum Einführen in den Körper des Patienten aufweist und an ihrem anderen Ende zum Anschließen an den Druckanschlussstutzen (64) der Verteilungsvorrichtung (2) ausgestaltet ist,
- eine Druckmessleitung (16) mit einem Conduit (20), der mit einem Drucksensor (18) ausgestattet und zum Anschließen an den Druckmessanschlussstutzen (66) der Verteilungsvorrichtung (2) gestaltet ist, und
- eine Spülleitung (22) mit einem biegsamen Conduit (26), der mit einer Tropfkammer (28) versehen und so gestaltet ist, dass sie an einem Ende an ein Spüllösungsreservoir (24) und am anderen Ende an den Spülanschlussstutzen (68) der Verteilungsvorrichtung (2) angeschlossen werden kann.
